# EUROPEAN PATENT APPLICATION

(11) **EP 2 308 400 A1**
(43) Date of publication of application: **13.04.2011**
(21) Application number: 10251763.8
(22) Date of filing: 07.10.2010
(51) Int. Cl.: A61B 17/34, A61B 17/32

(54) **Foam collar for surgical access devices**

(30) Priority: 07.10.2009 US 249297 P; 01.10.2010 US 895997
(71) Applicant: Tyco Healthcare Group LP, New Haven, CT 06511 (US)
(72) Inventor: Okoniewski, Gregory G., North Haven, CT 06473 (US)
(74) Representative: Soames, Candida Jane

(57) **Abstract**

A surgical access devices includes an access device and a balloon dissector assembly slidably mounted through the access device. The access device includes a cannula and an elongate collar affixed to the cannula. The balloon dissector includes a tubular member having a bore, an inflatable dissection balloon secured to a distal end of the tubular member, and an obturator slidably mounted in the bore of tubular member. The elongate collar includes a body portion which has a polyhedron prism shape and a tip portion. The body portion may extend from a surgical site inside the patient's body to the outside the patient's body. The elongate collar is made from a suitable foam or gel material having sufficient compliance to form a seal with the surgical objects and sufficiently compliant to accommodate off-axis motion of cannula during a surgical procedure.

## Description

### CROSS-REFERENCE TO RELATED APPLICATION

The present application claims the benefit of and priority to U.S. Provisional Patent Application Serial No. 61/249,297, filed October 7, 2009, the disclosure of which is herein incorporated by reference in its entirety.

### BACKGROUND

### Technical Field

The present disclosure relates to a surgical access device for use in endoscopic and laparoscopic surgical procedures, and more particularly, to a surgical access device having a foam collar for providing a seal.

### Background of Related Art

In laparoscopic and endoscopic surgical procedures, a small incision or puncture is made in a patient's body, e.g., in the abdomen, to provide an entry point for a surgical access device which is inserted into the incision and facilitates the insertion of instruments used in performing surgical procedures. When compared to the larger incisions typically found in traditional procedures, both trauma to the patient and recovery time are reduced for procedures involving small incisions. Surgical access devices typically include a cannula and a trocar. The cannula is utilized to provide an access port for surgical instruments and a conduit for introducing insufflation fluids into the body cavity. Typically, a trocar is positioned within the cannula. The trocar pierces tissue creating the incision and separates tissue allowing the cannula to be advanced towards the operative site. Upon placing of the cannula at the desired surgical site, the trocar is removed leaving the cannula in place. Thereafter, an insufflation fluid (e.g. carbon dioxide) is introduced into the body cavity to enlarge the area surrounding the target surgical site to create a larger, more accessible work area, prior to the introduction of surgical instruments into the patient's body. Accordingly, the maintenance of a substantially fluid-tight seal is desirable so as to prevent the escape of the insufflation gases and the deflation or collapse of the enlarged surgical site.

In order to maintain pneumoperitoneum and the cannula within the incision, it has been known to provide a balloon anchor and a foam collar on the cannula. The balloon anchor is disposed inside the body and inflated, which provides fixation of the cannula on the body and a seal which inhibits leakage of insufflation fluid. A foam collar is utilized on the exterior of the cannula to hold the cannula in place, in cooperation with the balloon anchor. When several cannulas are placed into a single incision, a gap may exist between the adjacent cannula tubes and permit the escape of insufflation fluids.

Accordingly, a continuing need exists to eliminate the gap created between several cannulas placed in close proximity of each other during a surgical procedure.

### SUMMARY

In accordance with the present disclosure, a surgical access device includes a cannula and an elongate collar disposed about a tubular member of the cannula. The surgical access device may also include a balloon dissector assembly slidably mounted through the tubular member of the cannula. The balloon dissector assembly includes a tubular member having a bore extending therethrough, an inflatable dissection balloon attached to a distal end of the tubular member, and an obturator slidably mounted in the bore of tubular member. The elongate collar may also be configured to extend from a surgical site inside of a patient's body to the outside of the patient's body. The tip portion may be axially tapered.

In accordance with another embodiment of the present disclosure, an elongate collar for use in a surgical access device includes a body portion having a polyhedron prism shape and a tip portion connected to a distal end of the body portion. In one embodiment, the elongate collar may be configured to extend from a surgical site inside of a patient's body to the outside of the patient's body. In addition, the tip portion of the elongate collar may have a radius dimension smaller than that of the body portion. Furthermore, the tip portion of the elongate collar is axially tapered.

### BRIEF DESCRIPTION OF THE DRAWINGS

Various embodiments of the present disclosure are described hereinbelow with reference to the drawings, wherein:

FIG. 1 is a schematic side elevational view of a surgical access device including an elongate collar in accordance with an embodiment of the present disclosure;

FIG. 2 is a perspective view of an obturator for use with the surgical access device of FIG. 1;

FIG. 3A is a perspective view of a cannula including an elongate collar in accordance with another embodiment of the present disclosure;

FIG. 3B is a perspective view of a cannula including an inflatable elongate collar in accordance with yet another embodiment of the present disclosure;

FIG. 4 is a top plan view of cannulas of FIG. 3 placed in close proximity to each other;

FIG. 5 is a partial side cross sectional view of the cannula of FIG. 3 placed in tissue; and

FIG. 6 is a top plan view of another embodiment of an elongate collar in accordance with the present disclosure.

### DETAILED DESCRIPTION OF EMBODIMENTS

Embodiments of the present disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farthest from the user while, the term "proximal," will refer to that portion of the instrument, apparatus, device or component thereof which is closest to the user. In the following description, well-known functions or constructions are not described in detail to avoid obscuring the present disclosure in unnecessary detail.

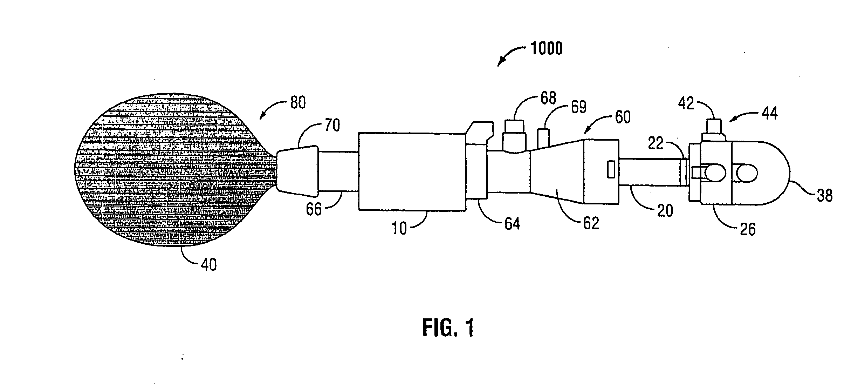

With reference to FIG. 1, a surgical access device 1000 having an elongate collar 10 is illustrated. An example of a surgical access device is disclosed in U.S. Patent Application Serial No. 12/244,024, filed October 2, 2008, the entire contents of which are incorporated by reference herein. While the following disclosure relates generally to the use of elongate collar 10 in combination with a balloon dissector assembly 80, it is also contemplated that elongate collar 10 of the present disclosure may be used with and not limited to, balloon retractors, balloon dissectors, or any other laparoscopic surgical instrument, to perform a variety of other surgical procedures known by one having ordinary skill in the art.

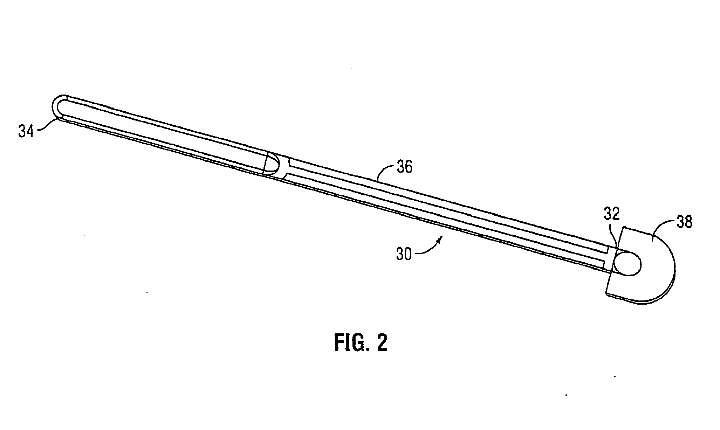

Referring additionally to FIG. 2, surgical access device 1000 is adapted for insertion within a tissue tract, e.g., through the abdominal or peritoneal lining, in connection with a laparoscopic or endoscopic surgical procedure. Surgical access device 1000 includes a cannula 60 and balloon dissector 80 slidably mounted through cannula 60. Balloon dissector 80 includes a tubular member 20 having a bore extending therethrough, and an obturator 30 slidably mounted in the bore of tubular member 20. Tubular member 20 has a housing 26 operatively connected to a proximal end 22 of tubular member 20. Obturator 30 includes a shaft 36 having a proximal end 32 and a distal end 34 having a blunt tip. A handle 38 is attached to proximal end 32 of shaft 36. Balloon dissector 80 further includes an inflatable dissection balloon 40 operatively secured to a distal end of tubular member 20 and in communication with the bore of tubular member 20. Inflatable dissection balloon 40 may have any shape and may be elastic or inelastic. As inflatable dissection balloon 40 is inflated in the tissue, balloon 40 causes the tissue to separate along a natural plane, providing an operating space. Balloon dissector assembly 80 further includes a balloon inflation port 42 and a valve assembly 44 connected to port 42. Valve assembly 44 couples with an inflation device (not shown) for transmission of inflation fluid to dissection balloon 40 through the bore of tubular member 20.

Cannula 60 includes a tubular member 66, a locking collar 64 operatively associated with tubular member 66, and elongate collar 10 extending distally from locking collar 64 and partially surrounding tubular member 66. Elongate collar 10 is affixed to locking collar 64 and is compressible against the abdominal wall to provide a seal. In particular, elongate collar 10 is configured to penetrate through tissue and is dimensioned to extend along the thickness of tissue, such that at least a proximal end portion of collar 10 extends out of the incision in tissue and at least a distal end portion of elongate collar 10 is exposed in the body cavity. Cannula 60 further includes a housing body 62 operatively connected to a proximal end of tubular member 66. Tubular member 66 has a tubular wall defining a passageway communicating with an opening in housing body 62 for receipt of operating instruments therethrough. Balloon dissector assembly 80 is supported on tubular member 66 and is in fluid communication with an inflation port 68 provided on housing body 62. A fluid channel (not shown) is defined within the wall of the tubular member 66 and connects inflation port 68 with balloon dissector assembly 80.

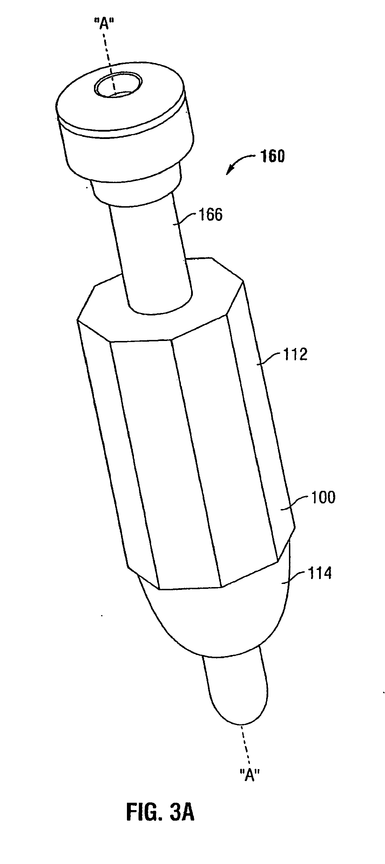

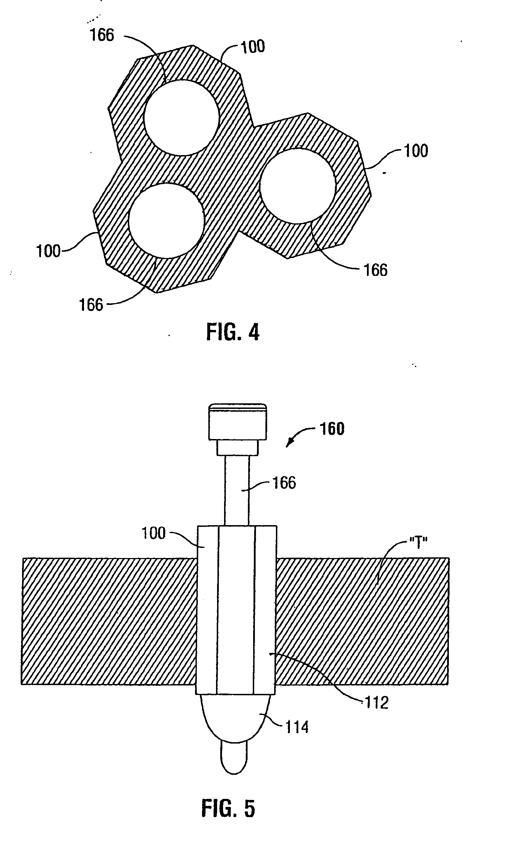

With particular reference to FIG. 3A, another embodiment of the present disclosure is shown generally as an elongate collar 100 defining a longitudinal axis "A-A." Elongate collar 100 includes a body portion 112 and a tip portion 114. Elongate collar 100 is affixed to tubular member 166 of a cannula 160, partially surrounding tubular member 166. Elongate collar 100 may be made from a compressible and/or flexible type material for example, but not limited to, a suitable foam or gel material having sufficient compliance to form a seal with surgical objects and also establish a sealing relationship with the incision site. Moreover, the foam or gel material is sufficiently compliant to accommodate off-axis motion of tubular member 166 during a surgical procedure. Elongate collar 100 is configured to penetrate through tissue "T" and is dimensioned to extend along the thickness of tissue "T." In particular, at least a proximal end portion of body portion 112 extends out of the incision in tissue "T" and at least a distal end portion of body portion 112 is exposed in the body cavity, as best shown in FIG. 5. It is also envisioned that a length of collar 100 is less than the thickness of tissue "T" such that the entire collar 100 is disposed within the incision through tissue "T".

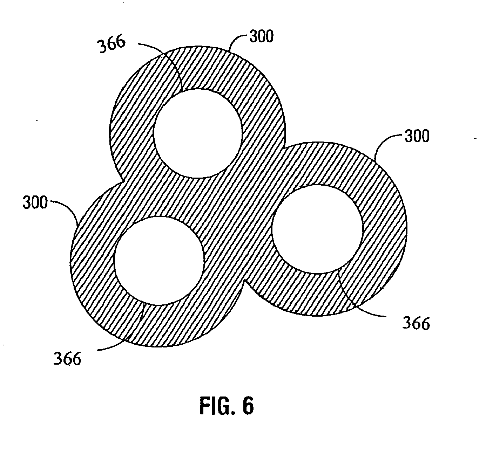

With particular reference to FIG. 4, elongate collar 100 enables the user to place a plurality of cannulas 160 through a single incision in tissue "T," while maintaining a substantially fluid-tight seal in tissue "T." Elongate collars 100 engage each other and compress as necessary to substantially eliminate any gap therebetween. Such configuration enables a number of cannulas 160 to be placed in close proximity to one another, while maintaining a substantially fluid-tight seal in the incision. Moreover, the shape of the cross section of elongate collar 100 may be tailored to meet the particular needs of a procedure being performed, for example, the number of cannulas 160 placed in tissue "T." Certain shapes of the cross section may provide better alignment with a particular number of cannulas 160 placed together. In this embodiment, each elongate collar 100 has a cross sectional shape of an octagon. However, such shape may be changed based on the application. For example, an elongate collar 300 may include a circular cross section, as shown in FIG. 6. However, regardless of the shape, each elongate collar is made from a material having sufficient compliance to form a seal with surgical objects and establish a sealing relationship with the incision site.

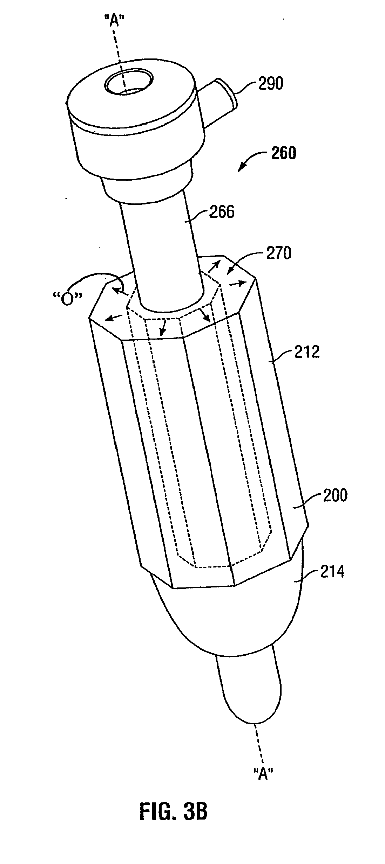

With reference now to FIG. 3B, an expandable or inflatable elongate collar 200 in accordance with another embodiment of the present disclosure is illustrated. Inflatable elongate collar 200 includes a body portion 212 and a tip portion 214. Body portion 212 includes a chamber 270, which may be expandable with supply of inflation fluid. Inflatable elongate collar 200 is affixed to tubular member 266 of a cannula 260, partially surrounding tubular member 266. Elongate collar 200 is configured to penetrate through tissue "T" and is dimensioned to extend along the thickness of tissue "T," such that at least a proximal end portion of body portion 212 extends out of the incision in tissue "T" and at least a distal end portion of body portion 212 is exposed in the body cavity, in a manner similar to that discussed hereinabove with respect to elongate collar 100. It is also envisioned that a length of collar 200 is less than the thickness of tissue "T" such that the entire collar 200 is disposed within the incision through tissue "T". Inflatable elongate collar 200 may be expandable with supply of inflation fluid through an inflation port 290 disposed adjacent a proximal end portion of tubular member 266. Upon supplying of the inflation fluid through inflation port 290, chamber 270 expands radially outward in the direction of arrows "O." (Uninflated elongate collar 200 is shown in phantom in FIG. 3B). Inflated elongate collar 200 may provide sufficient compliance to form a seal with surgical objects and establish a sealing relationship with the incision site. Inflated elongate collar 200 further accommodates off-axis motion of tubular member 266 during a surgical procedure, while maintaining a substantially fluid-tight seal against tissue "T."

A method of operation and use of surgical access device 1000 will now be described. First, a small incision is made in the skin of a patient, e.g., in the abdominal cavity wall, in close proximity to the umbilicus. A distal end of tubular member 66 is introduced into the incision while having obturator 30 placed within balloon dissector assembly 80. Obturator 30 is utilized to guide or advance cannula 60 into the tissue or abdominal wall. Once the distal end of tubular member 20 is positioned in the desired location in the body, obturator 30 is withdrawn from balloon dissector assembly 80. Elongate collar 10 which now extends from the surgical site inside the body to the outside the body seals the incision and anchors cannula 60 to the body. Then, inflatable dissection balloon 40 is inflated using known means, until the extraperitoneal space has been sufficiently dissected. Once the extraperitoneal space has been sufficiently dissected, dissection balloon 40 is deflated and removed. Thereafter, an insufflation fluid source is coupled or connected to an insufflation port 69. In this manner, insufflation fluid may be delivered to the extraperitoneal space to maintain the extraperitoneal space as desired. Moreover, endoscope (not shown), or other instruments may be introduced into the extraperitoneal space. With elongate collar 10 sealing the incision and anchoring tubular member 20 to the body, various surgical instruments may be introduced and withdrawn from the extraperitoneal space as needed and/or desired. Although the above procedure is disclosed with respect to surgical access device 1000, the principles are equally applicable to elongate collars 100, 200 and their respective cannulas 160, 260.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

## Claims

1. A surgical access device comprising:
a cannula having a tubular member;
an elongate collar affixed to the tubular member and extending along a portion of a length thereof; and
a balloon dissector assembly slidably mounted through the tubular member, the balloon dissector assembly including a tubular member having a bore extending therethrough, an inflatable dissection balloon operatively secured to a distal end ofthe tubular member, and an obturator slidably mounted in the bore of tubular member, wherein the elongate collar is configured to penetrate through tissue and extend along at least a portion of the thickness of tissue.

2. The surgical access device of claim 1, wherein the elongate collar includes a tip portion and a body portion having a plurality of planar faces.

3. The surgical access device of claim 2, wherein the tip portion of the elongate collar has a radial dimension less than that of the body portion.

4. The surgical access device of claim 2 or claim 3, wherein the elongate collar extends above a
surface of a patient's skin when the access device is inserted through an incision in the patient's skin.

5. The surgical access device of any of claims 2 to 5 wherein the tip portion is axially tapered.

6. A system for accessing a working space comprising:
a first access device and a second access device, each of the first and second access devices including:
a cannula having a tubular member extending therefrom; and
an elongate collar disposed about a portion of the tubular member, the elongate collar being deformable such that when the first and second access devices are abutting one another, the elongate collars deform and form a substantially fluid-tight seal therebetween.

7. The system of claim 6, further including a third access device having a cannula with a tubular member extending therefrom and an elongate collar disposed about a portion of the tubular member.
